# EUROPEAN PATENT SPECIFICATION

(11) **EP 0 934 352 B1**
(45) Date of publication and mention of the grant of the patent: **02.05.2007**
(21) Application number: 97911925.2
(22) Date of filing: 22.10.1997
(51) Int. Cl.: C08G 65/24, C08G 65/32, C08G 65/22, A61K 31/785

(54) **WATER-SOLUBLE POLYMERS FOR THE REDUCTION OF DIETARY PHOSPHATE OR OXALATE ABSORPTION**
WASSERLÖSLICHE POLYMERE FÜR DIE VERMINDERUNG DER ABSORPTION VON DIÄTETIKEM PHOSPHAT ODER OXALAT
POLYMERES SOLUBLES DANS L'EAU, QUI SONT UTILISES POUR REDUIRE L'ABSORPTION DE PHOSPHATE OU D'OXALATE ALIMENTAIRE

(30) Priority: 23.10.1996 US 28993 P
(43) Date of publication of application: 11.08.1999
(73) Proprietor: DOW GLOBAL TECHNOLOGIES INC., Midland, Michigan 48674 (US)
(72) Inventor: SIM0N, Jaime, Angleton, TX 77515 (US); MASTERSON, Tipton, Thomas, Lake Jackson, TX 77566 (US); STRICKLAND, Alan, D., Lake Jackson, TX 77566 (US); HILTON, Martha, L., Webster, TX 77598 (US)
(74) Representative: Raynor, John
(86) International application number: PCT/US1997/019322
(87) International publication number: WO 1998/017707

(56) References cited:
- EP-A- 0 081 291
- WO-A-95/05184
- DE-A- 2 437 927
- GB-A- 1 275 184
- US-A- 3 320 317
- US-A- 3 850 857
- US-A- 3 864 288
- US-A- 4 056 510

## Description

### FIELD OF THE INVENTION

This invention relates to a composition of matter comprising water-soluble polymers capable of complexing phosphate or oxalate, processes for preparing the polymers, processes for using the polymers in the complexation of dietary phosphate or oxalate in animals to prevent its absorption from the gastrointestinal tract, and formulations for their use as non-systemic agents.

### BACKGROUND AND SUMMARY OF THE INVENTION

It is known that levels of serum phosphate above the normal range have detrimental effects. Hyperphosphatemia, the condition of having excessive levels of phosphate in the serum, has been shown to cause pathological conditions such as osteodystrophy and secondary hyperparathyroidism [see, for example, M. E. Rubin *et al., Arch. Intern. Med*. 124, 663-669 (1969); and E. Slatopolsky *et al., Kidney Int'l*. 2, 147-151 (1972)]. The major group at risk for hyperphosphatemia is those patients who develop renal failure. Their hyperphosphatemia develops when their kidneys no longer function adequately to excrete the phosphate consumed in their diet and results in many complications. [See, for example, D. Mizumoto *et al., Clin. Nephrol.* 42, 315-321 (1994) for details of the clinical course.]

Treatment of patients with chronic renal failure is quite expensive and requires a great deal of time from the medical profession. Patients with renal failure cannot excrete all of the fluid, sodium, potassium, chloride, phosphate, nitrogen, and other minerals ingested in their diet and not needed in the body. Treatment for these patients progresses from minimal dietary restriction to severe dietary restriction to either peritoneal dialysis or hemodialysis as their renal status deteriorates. Renal transplantation may be required for many patients, but the lack of suitable donor kidneys may require the patient to undergo hemodialysis for years before transplantation is possible. Based on Medicare data, approximately 150,000 patients currently are receiving hemodialysis in the United States. By the stage of renal failure when dialysis is needed, many metabolic derangements are usually present. Since the kidneys are no longer handling the load of ingested fluid and electrolytes needing excretion, total body levels of sodium, potassium, calcium, phosphate, chloride, water, and various trace minerals are usually higher than normal. Excessive fluid retention and abnormal hormonal production causes hypertension. Abnormal metabolism causes hyperlipidemia and hypercholesterolemia. Consequently, patients on renal dialysis usually are receiving numerous medications to control their blood pressure, hormonal status, fat levels, and serum chemistries. They usually must also endure severe dietary restrictions including minimal protein intake, precise fluid restriction, strict sodium restriction, low fat intake, and high simple carbohydrate intake. These dietary restrictions are necessary because renal dialysis is not efficient in restoring body chemistries and resultant hormonal levels to normal levels. Dialysis frequently requires four to eight hours per session two to four times each week to remove the fluid, urea, creatinine, and electrolytes generated even by the restricted diet. Phosphate is particularly hard to control with dialysis since phosphate is poorly dialyzed by the membranes commonly used for dialysis.

Other diseases besides renal failure also cause hyperphosphatemia. Primary hypoparathyroidism is a rare cause of hyperphosphatemia. [See, for example, D. Mizumoto *et al., Clin. Nephrol*. 42, 315-321 (1994).] Poisoning with phosphate may also occur from administration of phosphate-containing enemas, oral purgatives, or urinary acidifiers. Thyroid carcinoma occasionally results in hyperphosphatemia. Rapid lysis of tumors during chemotherapy may also cause hyperphosphatemia which can be compounded by renal compromise from the excessive uric acid produced by the tumor lysis. [See, for example, T. Smith, *South. Med. J.* 81, 415-416 (1988).] Hyperphosphatemia has also been reported in infants of diabetic mothers. [See, for example, R. C. Tsang *et al., J. Pediatrics*. 89, 115-119 (1976).] Though much less common than renal failure, these diseases also cause significant health problems. Therapy for these causes of hyperphosphatemia frequently includes dietary restriction of phosphate to decrease the amount of phosphate absorbed.

Another disease state which causes significant morbidity and expense is the formation of renal stones. Renal stones cause 400,000 hospitalizations in North America each year. Oxalate stones cause 234,000 of these hospitalizations. Some mammalian metabolic pathways can result in the formation of oxalate which cannot be further metabolized and must be excreted through the kidneys. These pathways, however, account for less than a third of the urinary oxalate while dietary oxalate is the source of 67% of the urinary oxalate in metabolically normal patients [See, for example, R. P. Holmes, *et al., Scanning Microsco.* 9: 1109-1120 (1995)]. Both endogenous and dietary oxalate must be excreted through the kidneys along with other substances such as calcium, excess hydrogen, urea, and sodium. Calcium oxalate and oxalic acid have low solubility in urine and will easily precipitate to form renal stones. Patients with steatorrhea, ileal resection, ileal bypass, severe ileal mucosal disease, or pancreatic insufficiency have greater absorption of dietary oxalate than healthy persons and have greater problems with oxalate stones [See J. Q. Stauffer, *Am. J. Dig. Dis*. 22:921-928 (1977); A. F. Hofmann, *et al., Int. J. Obes.* 5: 513-518 (1981); K. Dharmsathaphorn, *et al., Dig. Dis. Sci.* 27: 401-405 (1982); *Gastroenterology* 84: 293-300 (1983); and D. P. D'Cruz, *et al., Br. J. Urol.* 64: 231-234 (1989)]. Genetically determined hyperoxaluria causes increased endogenous production of oxalate which can cause formation of renal oxalate stones. Dietary oxalate can exacerbate the renal stone formation in these patients.

Although the current therapy for hyperphosphatemia stresses dietary restriction of phosphate to decrease the phosphate load, this is often inadequate to completely treat the hyperphosphatemia and is quite bothersome to the patients. It usually becomes necessary to supplement dietary restriction with some therapy designed to prevent the phosphate which is ingested from being absorbed through the gastrointestinal tract. [See, for example, J. A. Ramirez *et al., Kidney Int'l.* 30, 753-759 (1986); and M. S. Sheikh *et al., J. Clin. Invest.* 83, 66-73 (1989).] Similarly, the treatments for hyperoxaluria have focused either upon decreasing the dietary intake of oxalate through elimination of various foods or upon preventing the absorption of oxalate from the gastrointestinal tract. Dietary restrictions have been difficult and confusing. Some authors suggest that all green vegetables, rhubarb, tea, and chocolate must be eliminated. Other authors add beets, nuts, wheat bran, and strawberries to the foods that must be restricted while allowing all green vegetables except spinach [See, for example, L. K. Massey, *et al., J. Am. Diet. Assoc.* 93: 901-906 (1993)]. Some authors suggest high calcium intake while others require strict limitations on calcium. Some authors require low protein diets while others insist that protein has no part in the treatment while dietary carbohydrates and fats must be kept to a minimum. Suggested intestinal binding agents for oxalate have included calcium, magnesium, aluminum, and fiber [See, for example, R. P. Holmes, *et al., Scanning Microsco*. 9: 1109-1120 (1995) and A. F. Hofmann, *et al., Int. J. Obes*. 5: 513-5180 (1981)]. Other authors fear that excessive calcium will lead to more stone formation. Some authors restrict fiber. None of these regimens has been particularly successful as evidenced by the 50% recurrence rate of renal stones within the first 6 years after removal of a renal stone. The preferred treatment would involve the binding of oxalate in the gastrointestinal tract by an agent that would prevent its absorption. The usual method of accomplishing this binding of either phosphate or oxalate involves the use of complexing agents.

"Complexing agents" are compounds which attract certain other compounds and hold them in association with the complexing agent. Many different mechanisms can operate to attract a target molecule or ion to a complexing agent. Simple complexing agents may be ions capable of reacting with a substance and forming an insoluble compound which then precipitates. This reaction of two ionic species to form an insoluble molecule is one of the simplest forms of complex formation.

"Chelants" are a type of complexing agent which form complexes known as "chelates". Chelants form two or more coordinate covalent bonds with other compounds, ions, or atoms through at least two sites in the complexing agent. These sites are frequently on "arms" that contain three to eight atoms, thereby allowing the formation of a ring of four to ten or more atoms when the complexed atom or molecule covalently binds to both ends of the chelating agent. Partially due to this ring formation, chelates are more stabile than compounds formed from the same two molecules but with only one coordinate covalent bond being formed. Stability of the chelate is also improved when several "arms" react, creating several rings. In addition to the stability from the increased number of rings, these compounds have increased stability from the steric interaction of the different arms which envelop the complexed atom or molecule thereby preventing easy dissociation from the complex.

Other forms of complexing agents include those which attract and hold molecules through ionic attraction. Dipole-dipole or dipole-ion attraction may also be the source of the complexing agent's ability to both attract and hold the complexed compound. Other forces which may be involved in helping complexing agents to function include hydrophobic and hydrophilic interactions.

These above-mentioned forces are given as purely illustrative examples and are not intended to be inclusive of all forces through which complexing agents can attract and hold compounds.

Functionalized solid resins have been used for complexation of various substances of biological interest. This is illustrated by cholestyramine, a crosslinked polystyrene with a portion of the styrene monomers functionalized with a quaternary amine chloride. This resin will attract and hold bile acids, thereby preventing their absorption from the gastrointestinal tract. [See "Questran^{™} Powder.", by Bristol-Meyers Squibb, Physicians Desk Reference, 51^{st} Edition, 1997; p 774-776.] However, cholestyramine suffers from being gritty, unpleasant in taste, and low in binding capacity. This requires that patients take large doses of a distasteful solid, which leads to poor patient compliance. Furthermore, cholestyramine exchanges its chloride for and then binds with-the bile acid ion. The amount of chloride released is frequently sufficient to cause metabolic acidosis at dosages of cholestyramine below those needed to treat the patient adequately. These problems of grittiness, unpleasant taste, low binding capacity, and ion exchange of an undesirable quantity of an ion from the resin are common to most resins investigated to date.

Therapy with complexation agents for hyperphosphatemia in patients with renal failure has focused on the precipitation of phosphate in the gastrointestinal tract with salts of either aluminum or calcium. Aluminum salts (usually the hydroxides, such as Amphojel^{™} by Wyeth-Ayerst or Maalox^{™} by Ciba) have been unsatisfactory because aluminum was absorbed from the gastrointestinal tract and caused osteomalacia and neurological disease. The carbonate salt of calcium (Tums^{™} by SmithKline Beecham) has been the most widely clinically used agent although the acetate (PhosLo^{™} by Braintree), the citrate, and the alginate salts have also been used. These agents result in excessive absorption of calcium with resultant soft tissue calcification. Recently, calcium β-hydroxy-β-methylbutyrate has been proposed as a phosphate complexing agent. [See, for example, M. F. Sousa *et al., Nephron.* 72, 391-394 (1996).]. This salt also works through precipitating calcium phosphate, resulting in all the problems associated with the other calcium salts. It has been proposed for renal dialysis patients mainly because the β-hydroxy-β-methylbutyrate is reported to improve protein metabolism. Anion exchange resins have been compared in *vitro* with aluminum salts. Bio-Rex^{™} 5, Dowex^{™} XF 43254, Dowex^{™} XY 40012, and Dowex^{™} XY 40013 all had binding capacities about half that of aluminum compounds. Dowex^{™} SBR and Dowex^{™} 1-X8 could bind only a third of the phosphate that aluminum salts bound. Dowex^{™} XF 43311 and Dowex^{™} XY 40011 could bind 80% of the phosphate that aluminum salts could bind. (All Dowex resins are by The Dow Chemical Company and are strong base anion exchange resins based on quaternary amine functionality.) [See, for example. H. M. Burt *et al., Uremia Invest.* 9(1), 35-44 (1985-1986); and H. M. Burt *et al., J. Pharm. Sci.* 76(5), 379-383 (1987).] These agents have not been used in patients because they release chloride which could cause acidosis, they require large doses to compensate for the low binding capacity, and they bind bile acids, which could limit the permissible dosage before diarrhea occurred from fat malabsorption. Other complexing agents for phosphate have been proposed. These have included iron salts, crosslinked iron dextran, rare earth salts, and zirconyl chloride. [See, for example, K. Spengler *et al., Nephrol., Dial., Transplant*. 11, 808-812 (1996); and L. Graff *et al., Res. Commun. Mol. Pathol. Pharmacol.* 90, 389-401 (1995).] Each of these agents is designed to complex phosphate by forming a precipitate between the metals and the phosphate. None of these agents has been administered to human volunteers or to patients. The amount of activity in searching for phosphate complexing agents is testimony to the need for a better method to treat hyperphosphatemia than is currently available in either dietary restriction or known and available medications.

### Summary of the Invention

Surprisingly, it has now been found that, in contrast to water-insoluble resins and polymers for use as *in vivo* phosphate or oxalate reduction agents, it is now possible to use a water-soluble polyether glycol polymer. This polymer has a structural backbone of carbon atoms and oxygen atoms where there are at least two consecutive carbon atoms present between each oxygen atom. Examples of such polymers include the polyethylene glycols and polypropylene glycols. These polymers must be water-soluble and have a moiety on the backbone of the polymer or a functionalized derivative on the polymer which is cationic at physiological pH and permits complexation with phosphate or oxalate. These polymers have an average molecular weight from about 5,000 to about 750,000 Daltons. These polymers are formulated in conventional manners and used *in vivo* in an animal to reduce the amount of phosphonate or oxalate present. To prepare these polymers, caution must be used to obtain the desired molecular weight and solubility such that these polymers are often functionalized with derivatives.

### Detailed Description of the Invention

Accordingly, the present invention relates to a series of water-soluble polyether glycols (PEG) that are capable of avoiding the problems with the current and proposed phosphate or oxalate complexing agents. The PEG includes polyepihalohydrin (PEi) polymers where the halo portion of the PEi polymer can be either chloride, bromide or iodide. The polyether glycols (PEG) have a structural backbone of carbon and oxygen where the number of consecutive carbon atoms must be two or more and there are no consecutive oxygen atoms. Examples of these polyether glycols are polyethylene glycol and polypropylene glycol. Water-solubility of these present PEG polymers, which are usually derivatives, leads to a homogenous mixture with the biological fluids being treated in the animal (meaning warm-blooded mammals, including humans) rather than the slurry of an insoluble resin in the biological fluids as known from present methods. It has been discovered that this solubility results in better mixing and improved complex formation which allows lower doses of complexing agent to be used. Furthermore, administration of the agent is more pleasant to the animal since the gritty texture is not present and since the taste of the agent is decreased and can be more completely masked with an aqueous flavor than could the resin.

The formulation which can be use with the PEG-D (polyether glycol derivatives) polymers, especially the PEi-D polymers, is for non-systemic use. Thus, these formulations are administered orally to the animal. The dose of the PEG-D polymer is effected by the amount of phosphate or oxalate that must be removed.

A phosphate binder given by mouth would be dosed according to a ratio of binding site on the polymer to phosphate in the diet. The normal American diet has 48 to 65 mmol of phosphorus per day. A 1X load would be one mole of polymer binding sites per mole of dietary phosphate. A 5X load would be 5 moles of polymer binding sites per mole of dietary phosphate.

PEi / TMA (14,000 M_{w}) at pH 7, in saline, and with a 5X load of polymer absorbed 0.69 mmol phosphate per gram, binding about 98% of the phosphate. To absorb 48 to 65 mmol of phosphate would require 70 to 94 grams of this polymer per day. PEi / EDA (about 14,000 to 20,000 M_{w}) at pH7, in saline, and with a 5X load of polymer absorbed 1.38 to 1.73 mmol phosphate per gram (about 98% of the phosphate) and would require.28 to 47 grams per day to bind all the phosphate in the diet.

In rat trials, a 1X load was quite effective in lowering serum phosphate within a week or two. A 2X dose lowered the serum phosphate faster. A 5X dose lowered the serum phosphate within a few days, but the rats were not eating normally, so some of the decrease in phosphate may have been the result of starvation. From the rat trials, it would appear that a usual dose would be 0.5X to 1X load, while doses as high as 5X could be used for a day or two to quickly lower phosphate. Thus, the usual dose would be from about 3 to about 10 grams per day (or about 1 to about 3 grams per meal, 3 meals per day), and short term dosing could be as high as about 15 to about 50 grams per day (or about 5 to 15 grams per meal, 3 meals per day). Therefore the effective amount of the PEG-D or PEi-D is from about 1 to about 15 grams per meal for removal of phosphate from the diet.

The normal American diet varies from 0 to 300 mg of oxalate (0 to 3.3 mmol) per day. Since the formula weight of phosphate and oxalate are roughly equal, while the amount of oxalate in the diet is roughly 5% that of the amount of phosphate in the diet, a starting dose would be from about 0.6 to about 2 gram per meal, 3 meals per day. Therefore the effective amount of the PEG-D or PEi-D is from about 0.6 to about 2 grams per meal.

The formulations for administrating the PEG-D polymers or the PEi-D polymers of this invention are any suitable oral formulations, including but not limited to solid dosage forms such as tablets, capsules, caplets, gelcaps, dry powders, dry granular mixes, and other solid formulations; and liquids such as suspensions, solutions, and liquid mixes with commercially available juices, breakfast drinks, and fruit drinks. Customarily, pharmaceutically-acceptable carriers are present in the formulation. Thus one or more of the following items are present: excipients; binders such as starch, polyvinyl pyrrolidone (PVP) and pregelatinized starch; lubricants such as magnesium stearate, calcium stearate, and stearic acid; and other inert ingredients, including flavorings, preservatives, buffers, anti-caking agents, opacifiers, sugars such as sucrose and synthetic sweeteners, edible oils such as mineral oils, and colorants, can be present in the formulation with PEG-D. Any edible formulation commonly employed in food, beverage or drug substances may be employed as a formulation in a conventional manner. The final formulations are prepared by methods known in the art.

It was also determined that in order to prevent absorption of phosphate or oxalate from the gastrointestinal tract and minimize adverse gastrointestinal side effects, the PEG-D polymer or PEi-D polymer as a complexing agent should be larger than about 5,000 Daltons and preferably larger than about 10,000 Daltons. However, polymers of extremely high molecular weights may no longer be soluble in water. [See Finch, C. A., "Chemical modification and some cross-linking reactions of water-soluble polymers", Chemistry and Technology of Water-Soluble Polymers, Finch, C. A., ed., Plenum, New York, NY, 1983; pp 81-111.] The molecular weight range where these changes occur depends on the specific PEG or PEi-D polymer being considered, but loss of water-solubility generally occurs above about 750,000 Daltons. Loss of water- solubility makes the PEG or PEi-D polymer less palatable for patients and less effective in binding phosphate or oxalate. The present invention represents a significant improvement over all other known or available agents for the removal of phosphate or oxalate from the gastrointestinal tract due to the water-solubility of the PEG or PEi-D polymers of the invention, their polymeric nature, and the lack of need for a metal ion designed to precipitate phosphate or oxalate.

Many water-soluble polymers are known, and higher molecular weight polymers are usually less soluble in water than lower molecular weight polymers of the same composition. [See Thomson, R. A. M., "Methods of polymerization for preparation of water-soluble polymers", in Chemistry and Technology of Water-Soluble Polymers. Finch, C. A., ed., Plenum, New York, New York, 1983; pp 31-70; and Fuchs, O., "Solvents and non-solvents for polymers", Polymer Handbook, 3^{rd} Edition, Brandrup, J. and Immergut, E. H., eds. Wiley, New York, New York, 1989; pp VII/379 - VII/402.]

The water-soluble polymers of this invention are amine derivatives of polyethylene glycols (PEG-D). These polymers can be prepared by polymerizing an epihalohydrin followed by derivatizing the resulting polyepihalohydrin to provide the polyepihalohydrin derivative (PEi-D) polymer. (The conditions for preparation of this PEi-D polymer are provided later.) Current industrial methods of producing polyepihalohydrins either produce short polymer chain lengths with the average molecular weight range below about 3,000 or molecular weight ranges greater than 1,000,000. [See E. J. Vandenberg, *J. Polym. Sci.* 47, 486-489 (1960);: Vandenberg, E. J. "Elastomers, Synthetic (Polyethers)", Kirk-Othmer Encyclopedia of Chemical Technology, Third Edition. Volume 8. Kroschwitz, J., ed. Wiley, New York, New York, 1979; pp 568-582; and Owens, K., Kyllingstad, V. L. "Elastomers, Synthetic (Polyethers)", Kirk-Othmer Encyclopedia of Chemical Technology, Fourth Edition, Volume 8, Kroschwitz, J., ed., Wiley, New York, NY, 1993; pp 1079-1093.] Therefore, this invention also provides the process of producing polyepihalohydrin amine derivative (PEi-D) polymers in the range of 5,000 to 750,000 Daltons. These PEi-D polymers are particularly well suited for use in the prevention of absorption of dietary phosphate or oxalate from the gastrointestinal tract.

This invention pertains to water-soluble PEi-D polymers which are capable of complexing phosphate or oxalate and their use in decreasing absorption of dietary phosphate or oxalate, respectively, from the gastrointestinal tract. Such PEi-D polymers can be described based on the backbone of the polymer, the substituents attached to the backbone, the functional groups which improve water-solubility, and the functional groups which permit complexation of phosphate or oxalate.

The water-soluble complexing PEi-D polymers of the present invention comprise polymers having a backbone structure that either provides water-solubility and phosphate or oxalate complexing ability, or that allows side chains which permit water-solubility and functionalization to permit complexation of phosphate or oxalate ,and which preferably have an average molecular weight of from about 5,000 to about 750,000 Daltons, and more preferably from about 10,000 to about 80,000 Daltons. Water-solubility of the PEi-D polymers of this invention is defined as the ability of the polymer to form a homogeneous mixture of an efficacious quantity of the polymer with water. Preferably, water-solubility of the PEi-D polymers of the present invention would imply at least 0.01 gram (g) of the polymer would dissolve in 1000 milliliters (mL) of water, and more preferably, at least 1 g of the polymer would dissolve in 1000 mL of water. Decreasing phosphate or oxalate absorption from the gastrointestinal tract indicates that the percentage of dietary phosphate or oxalate removed from the gastrointestinal tract by absorption into the body is less when the PEi-D polymers of this invention are used, than it is when the polymers are not used. This decrease can be determined by comparison of the percentage of dietary phosphate or oxalate in the feces of a animal while the animal is ingesting the PEi-D polymer with the same percentage when the animal is not ingesting the polymer or any other phosphate- or oxalate-complexing agent. Appropriate consideration of changes in phosphate or oxalate absorption during growth can be accomplished by paired studies of control animals. Further corroborating data for the decrease in gastrointestinal phosphate or oxalate absorption from an animal may be obtained from comparison of urinary phosphate or oxalate excretion as a percentage of dietary phosphate or oxalate before and during an oral trial of the polymer that lasts for over a few weeks since the urinary phosphate or oxalate excretion will decrease when the amount of absorbed phosphate or oxalate is not sufficient to maintain phosphate or oxalate homeostasis with the normal urinary phosphate or oxalate excretion. An additional corroboration of the decrease in gastrointestinal absorption of phosphate or oxalate can be obtained by measuring serum levels of the species before and during administration of the polymer.

Examples of polymers which are included in this invention are water-soluble polymers which have a backbone of polyethylene glycol derivatized with functional groups (PEG-D) that improve water-solubility and phosphate or oxalate complexing ability. Some of these polymers might require side chains with functional groups that allow water-solubility or complexation of the phosphate or oxalate. The present invention includes both of these sets of derivatized polymers (PEG-D). Examples of side chains which could improve water-solubility, phosphate or oxalate complexing ability, or both include connecting to the polymer backbone, either directly or through C₂-C₆ alkylene or (C₂-C₆ alkyl)aryl groups, such functional groups as hydroxyl groups, sulfonates, phosphonates, nitro groups, amine groups, phosphine groups, carbonyl groups, thiol groups, halides, and combinations of these groups. These examples of polymer side chains are given as examples only and are not intended to limit the side chains or the functional groups on the polymers of this invention. In general, it is preferred that the polymers of this invention have as small a formula weight as possible for the monomer unit of the polymer in order to decrease the dosage for the animal.

One technique for preparing a polyethylene glycol backbone (PEG) is the polymerization of an epihalohydrin, such as epichlorohydrin, in the presence of a Lewis acid of moderate strength in a solvent that will not act as a chain terminator. Dichloromethane is an example of such a solvent, whereas alcohols or solvents containing water would not be preferred. These techniques are generally known in the art, see for example, U. S. Patent 2,871,219; or E. J. Vandenberg, *J. Polymer Sci.* 47, 486-489 (1960). This particular technique has the advantage of preparing the polyethylene glycol backbone with a functionalized side chain (*i*.*e*., CH₂Cl) from the backbone that allows easy substitution of other functionalities as will be described below. Another monomer that may be used in similar reactions to create a polyethylene glycol backbone with functionalized side chains is 3,4-dichloro-1,2-butane oxirane. Other methods of preparing a polyethylene glycol backbone with side chains from the backbone to allow further functionalization of the polymer are also included in this invention. These methods include reactions on previously formed polyethylene glycol to dehydrogenate the carbon-carbon bond and then introduce functionality across the double bond. A preferred starting material for a polyethylene glycol backbone is an epihalohydrin such as epichlorohydrin or epibromohydrin.

As previously explained, it is desirable for the polymers of the present invention to be water-soluble. Some polymer backbones contribute to solubility in water. The oxygen atoms in the backbone of various polyethylene glycols improve water-solubility. Some polymers may benefit from functionalization of side chains to promote water-solubility. Functionalization of the polymer backbone to improve water-solubility may be done by placement of groups which permit hydrogen bonding to water or ionic dissociation in water. Such groups include hydroxyl groups, amine groups, sulfonate groups, phosphonate groups, carbonyl groups, carbamate groups, nitro groups, and carboxylic acid groups. These examples are intended only as examples of functional groups which might improve water-solubility and are not intended to limit the functional groups of this invention. Inclusion of these groups as functional groups of the polymers can be done by having the groups already in the monomer when the polymer is prepared or by a separate reaction to introduce the group to a polymer. The former technique is demonstrated by preparation of polyvinylsulfonic acid and polyacrylic acid. This technique is well known in the art of polymerization.

The second technique involves introduction onto the polymer of the desired functionality based on transformation of the preexisting functionality of the polymer. Such transformations of functional groups are known in the art of organic chemistry. For example, Comprehensive Organic Transformations: A Guide to Functional Group Preparations, by Richard C. Larock presents many preparative routes for the introduction of various functional groups. This reference includes tables which list the desired functionality, the present functionality, and the reaction sequences which have been reported to accomplish the transformation. Other sources of preparative techniques include Advanced Organic Chemistry: Reactions, Mechanisms, and Structure, Fourth Edition, by Jerry March; Nitration: Methods and Mechanisms by George A. Olah, Ripudaman Malhotra, and Subhash C. Narang; and Advanced Organic Chemistry by Francis A. Carey and Richard J. Sundberg, Plenum Press, NY, 1990.

Additionally, the polymers of the present invention possess the ability to complex with phosphate or oxalate, as explained above. To do this, it is preferred that the polymer backbone either contain a moiety or be functionalized with a moiety which will permit complexation with phosphate or oxalate. Any moiety which will be cationic at the physiological pH (about pH 6.5 to 7.5) to which it is exposed will generally facilitate complexation with phosphate or oxalate. Amines and phosphines are examples of such moieties which may be cationic at physiological pH. To complex with phosphate or oxalate, amines should either be quaternary amines or be capable of being converted to quaternary amines under physiological conditions. Similarly, phosphines should either be quaternary phosphines or be capable of easy conversion to quaternary phosphines under physiological conditions in order to be cationic. Thus, the amines can be primary, secondary, tertiary, and quaternary amines or polyamines. More preferred functionalities include those selected from the group consisting of ammonia, ethyleneamines, alkanol amines and (C₁-C₁₀ alkyl)amines. Preparative reactions to introduce these groups can be found in the same references mentioned above for functional groups designed to improve water-solubility.

Thus, the polymers of the invention (PEG-D) can be prepared in either one or two steps.

One Step: Water-soluble phosphate-complexing polymers or oxalate-complexing polymers may be prepared in one step when the monomer contains appropriate functionality to allow polymerization that produces an appropriate backbone and simultaneously produces side chains with functionality that can complex with phosphate or oxalate. Either the backbone, the side chains, or both would result in solubility in water.

Two Step: In the two-step process, the first step involves the preparation of a backbone with appropriate leaving groups. These leaving groups are replaced in the second step to introduce the desired functionality needed to improve water-solubility, to improve complexation ability, or both.

Another aspect of the present invention is the use of these PEG-D or PEi-D polymers as non-systemic agents in the prevention of absorption of dietary phosphate or oxalate in the gastrointestinal tract. For this application it was discovered that water-solubility and size both play important roles. As described above, water-solubility improves the mixing of the complexing agent with the target compound which leads to more effective complexing. Furthermore, water-solubility makes the agent more palatable thereby increasing patient compliance. Size of the molecule in this type of application is important since molecules less than about 1,500 Daltons can be absorbed from the gastrointestinal tract into the bloodstream, which is not desirable for the present invention. Molecules between 1,500 and about 5,000 Daltons are not absorbed from the gastrointestinal tract but may cause an osmotic effect which draws water into the intestine and causes diarrhea and possible dehydration. Water-solubility generally decreases with increasing size of the polymer. Because of this, there may be an upper molecular weight limit of about 750,000 Daltons for the polymers of this invention in addition to the lower limit on molecular weight described above.

For some polymers, backbones of the appropriate length can be achieved using means known in the art. For instance, polyvinylpyrrolidone of the appropriate molecular weight is obtained by polymerizing vinylpyrrolidone followed by separation of the resulting mixture of molecular weights through either size exclusion membranes or preparative size exclusion chromatography. Other polymers may be prepared in the correct molecular weight range by judicious choice of the ratio of moles of monomer to moles of catalyst in the starting reaction mixture. However, some polymers are difficult to prepare in the preferred weight range. These polymers usually require such vigorous catalysts to start the polymerization that only very short polymers are made before side reactions stop the polymerization. When the catalysts for these polymerizations are partially inactivated in an attempt to permit better control of the degree of polymerization, the reaction proceeds to extremely large molecular weights without the ability to control the degree of polymerization. These issues are well known in the art and are discussed in Allcock, H. R. and Lampe, F. W., Contemporary Polymer Chemistry, Second Edition, Prentice Hall, Englewood Cliffs, New Jersey, 1990, pages 21-333; and in Young, R. J. and Lovell, P. A. Introduction to Polymers, Second Edition, Chapman and Hall, New York, 1991, pages 15-133. Separation techniques, such as those described for polyvinylpyrrolidone, may be successful in processing polymers with high degrees of polymerization and isolating those polymers with lower molecular weights.

One embodiment of this invention is that of polyepichlorohydrin polymers (PEi-D polymers) which have a molecular weight of 5,000 Daltons and greater, more preferably at least 12,000 Daltons, still more preferably at least 15,000 Daltons. The polymers of this invention can be any molecular weight above these minimums, but are preferably less than 750,000 Daltons, more preferably less than 500,000, most preferably less than 300,000 Daltons, and most especially preferably less than 80,000 Daltons. Generally, polyepichlorohydrin polymers have not been prepared in the preferred molecular weight range. Many polyepichlorohydrin polymers reported in the prior art were too low in molecular weight, usually being below 3,000 Daltons. [-See T. Aida *et al., Macromolecules* 21, 1195-1202 (1988); A. Le Borgne *et al., Makromol. Chem., Macromol. Symp*. 73, 37-46 (1993); and R. Nomura *et al., J. Polym. Chem.* 26, 627-636 (1988).] These polymers were usually made with catalysts that were very strong, such as alkyl aluminum or boron compounds. When oxygen containing compounds were added to the aluminum catalysts to partially inactivate them, polyepichlorohydrin that had molecular weights over 1,000,000 Daltons resulted. [See U. S. Pat. No. 2,871,219; E.J. Vandenberg, *J. Polymer Sci.* 47, 486-489 (1960); and J. Wu *et al., Polym. J.* 22, 326-330 (1990).]

It was discovered in the present invention that the appropriate weight range of polyepichlorohydrin could be made using catalysis by triethyloxionium hexafluorophosphate or by 1,2-ethyl di(trifluoromethanesulfonate) - *i. e.* "1,2-ethyl ditriflate." Triethyloxionium hexafluorophosphate has been reported as a catalyst capable of polymerizing epichlorohydrin by adding epichlorohydrin groups to each end of a central ethylene glycol to make molecular weights between 900 and 1000 Daltons. [See Okamoto, Y., "Cationic ring-opening polymerization of epichlorohydrin in the presence of ethylene glycol", Ring-opening Polymerization: Kinetics, Mechanisms, and Synthesis, McGrath, J. E., ed., ACS, Washington, D. C. 1985; 286:361-372.] The present invention relates to the preparation of polyepichlorohydrin without the presence of ethylene glycol. The present invention has produced appropriate molecular weight polyepichlorohydrin through control of polymerization terminating reactions. This control was obtained by careful distillation of all the reactants and solvents to exclude water, careful control of the temperature during the exothermic reaction, and judicious control of the ratio of catalyst molecules to epichlorohydrin molecules at the start of the reaction. Continuous addition of epichlorohydrin to the reaction mixture after a set number of growing polymers have been initiated also allowed optimal control over the molecular weight of the polymer. Another method of preparing the desired molecular weight of polyepichlorohydrin is the use of 1,2 ethyl ditriflate as a catalyst. A third method of producing polyepichlorhydrin with molecules in the appropriate weight range is the use of fluoboric acid as a catalyst with appropriate control of temperature and addition rates.

When it is needed to improve the water-solubility, the complexing ability, or both of the polymer, placement on the backbone of various functionalities such as amines, aminocarboxylates, crown ethers, azamacrocycles, or carboxylates, can be accomplished in a second step. The choice of the functionality is made depending upon the desired activity of the resultant water-soluble complexing polymer. Preferably, the desired functional groups will complex phosphate or oxalate on a one mole of phosphate or oxalate to one mole of monomer complexation site basis and will permit quantities of polymer needed for individual doses, such as 1 to 10 grams, to be soluble in small quantities of water, such as 1 to 8 ounces. Ideally, one functional group would perform both of these tasks, but placement of two or more different functional groups on the polymer backbone may be needed. Placement of the desired complexing group or groups on a polyethylene glycol backbone is performed by the appropriate reactions depending upon the functionality that is on the polymer after synthesis and the identity of the desired complexing agent. In a preferred embodiment of the invention where the polymer backbone is prepared from epichlorohydrin, functionalization of the polyepichlorohydrin is performed by reacting it under nucleophilic conditions with an appropriate amine to provide the reactivity needed for the desired use of the water-soluble chelating polymer. For example, if the situation only requires binding phosphate in an acid environment, then the acid can protonate an amine to an ammonium which, being positively charged, will bond to an anion like phosphate or oxalate. Thus, using a primary or secondary amine (such as ethylenediamine, diethylenetriamine as either the free material or with the primary amines blocked to force substitution at the secondary nitrogen, or higher analogs of ethyleneamine) will suffice, with one nitrogen displacing the chlorine while the other nitrogen remains free to be protonated and bind the anion. Even ammonia substitution for the chlorine atom could provide an amine capable of being protonated. If, on the other hand, it is necessary to bind the phosphate even in alkaline conditions (such as in the case of binding phosphate in the gastrointestinal tract of a patient who is on Tagamet^{™} so that there is no stomach acid present), a tertiary amine, such as trimethylamine, may replace the chlorine atom. This would result in a quaternary ammonium compound which is positively charged regardless of the pH. Thus, for example, a polymer of the formula: 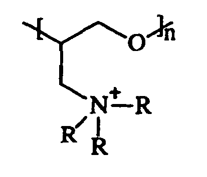 where each R independently may represent hydrogen; an unsubstituted C₁-C₆ alkyl group which may be unbranched, branched, or cyclic; a substituted C₁-C₆ alkyl group which may be unbranched, branched, or cyclic; an unsubstituted C₆-C₁₄ aryl group; a substituted C₆-C₁₄ aryl group; or 1 or 2 R groups may be absent, (*e*.*g*., where only one of the R groups may be absent) as in the case when the nitrogen depicted has only three substituents (including the connection to the polymer backbone) rather than four substituents would be one example of the polymers of this invention. For example, when ethylenediamine is substituted onto polyepichlorohydrin, the formula would have one R group as hydrogen, one R group as an aminoethyl group, and one R group absent. In another example, trimethylamine is substituted onto polyepichlorohydrin resulting in the above formula having each of the three R groups be a methyl group. In a further example, hexadecylamine is substituted onto polyepichlorohydrin resulting in one R group being a hexadecyl group, one R group being a hydrogen, and one R group being absent.

When high selectivity or high stability constants for the complex are needed, the polyepichlorohydrin or other water-soluble polymer may be substituted with a macrocyclic compound with oxygen, nitrogen, sulfur, or a combination of these as the heteroatoms in the macrocycle such as crown ethers, azacrown ethers, thiocrown ethers, cyclodextrins, or porphyrins. [See for example, R. M. Izatt *et al., Chem. Rev.* 91, 1721-2085 (1991); and S. Tamagaki *et al., Supramol. Chem*. 4, 159-164 (1994).] In cases where macrocyclic complexing groups are substituted onto the polymer, other functionality may also be required to insure solubility in water.

The PEi polymers are derivatized to form their corresponding PEi-D polymers in the manner discussed above. When the amine group is desired as the functionalized group in the derivative, the PEi may be reacted in the neat amine solvent. Usually a minimum of a four molar excess, preferably from 12 to 16 molar excess, of amine to the chloromethyl group in the PEi is used. An exception to this molar requirement was trimethylamine where as little as 0.5 mole of the amine to one mole of PEi was required in a 20% aqueous solution. Water is usually kept out of the reaction system during this step because water contributes to hydrolysis of the chloromethyl groups in the PEi. The temperature range for the reaction is from about 25 to about 120°C. The rest of the reaction was run as described above and the examples. The conversion of the chloride in the chloromethyl group in polyepichlorohydrin to the amine derivative is from about 10 to about 80%.

The invention will be further clarified by a consideration of the following examples, which are intended to be purely exemplary of the present invention.

### General Experimental Procedures

A. Procedure for determining the amount of amine added to the polyepichlorohydrin (PEi).
The amount of functionalization of ethylenediamine (EDA) on a polyepichlorohydrin polymer was determined by a copper titration method. The PEi/EDA solution was titrated with a copper chloride solution in the presence of a Murexide^{™} indicator. The copper was chelated by the EDA until saturation at which point the excess copper complexed the indicator and this end point was observed using a colorimetric detector.
Solutions needed for the METTLER^{®} DL40GP MemoTitrator:
1. A 0.01 M copper chloride solution prepared by adding 1.705 g (0.01 moles) of cupric chloride {CuCl₂ • 2H₂O} [from Fisher](FW170.48) to a one liter volumetric flask and diluting to the mark with deionized water.
2. A 0.002M sodium acetate buffer solution prepared by adding 0.272 g (0.002 moles) of sodium acetate, trihydrate {CH₃COONa • 3H₂O} [ from Fisher] (FW136.08) to a one liter volumetric flask and diluting to the mark with water.
3. A 0.1% Murexide^{™} indicator solution prepared by adding 5.0 g (0.0176 moles) of ammonium purpurate acid [from Fisher] (FW284.19) to a 500 mL volumetric flask and diluting to the mark with water.

Specialized 125 mL, disposable, polyethylene sample beakers (made to fit a METTLER^{®} ST20 sample changer) were tared on a METTLER^{®} AE 163 balance and loaded with an aqueous solution of PEi / EDA (an amount estimated to deliver -8 mg of PEi / EDA). The weight of this sample was recorded automatically in method 365 of a METTLER^{®} DL 40GP MemoTitrator. To this PEi/ EDA solution was added 80 mL of de-ionized water, 4.0 mL of a 2 mMolar aqueous solution of sodium acetate, and 0.5 mL of a 0.1 % aqueous solution of ammonium purpurate acid (Murexide^{™} indicator). The sample solutions were placed on the sample changer and titrated with 0.01 M copper chloride solution. The end point was observed using a METTLER^{®} DP550 Phototrode colorimetric detector and entered into the MemoTitrator. The amount of functionalization on the poly-epi polymer could then be calculated based on the number of moles of copper chelated by the EDA. An example of this titration method can be seen in Table I below:

| **Table I. Data from reacting Ethylenediamine with Polyepichlorohydrin** | | | | | | | |
|---|---|---|---|---|---|---|---|
| | | | | | | | |
| Reaction # | EDA:Polyepi mole ratio | Temp (°C) | Dialyzed thru MwCO | Solution Conc. (%) | Sample weight (g) | Amount of titrant (mL) | Percent of EDA added to Polyepi |
| 53554-40a | 16 | 108 | 1,000 | 6.6 | 0.1254 | 2.460 | 34.54 |
| 53554-40b | 16 | 108 | 3,500 | 7.1 | 0.1123 | 2.320 | 33.80 |
| 53554-40c | 16 | 108 | 12-14,000 | 3.6 | 0.2064 | 2.203 | 34.45 |
| 53554-41a | 4 | 25 | 1,000 | 10.8 | 0.0680 | 0.735 | 11.63 |
| 53554-41 b | 4 | 25 | 3,500 | 10.2 | 0.0800 | 0.800 | 11.39 |
| 53554-41c | 4 | 25 | 12-14,000 | 7.2 | 0.1132 | 0.842 | 12.00 |

From the data in Table I above, it is evident that the more ethylenediamine (EDA) used as a reactant with the polyepi polymer (PEi) and the higher the reaction temperature, the greater the amount of EDA added to the polymer backbone. Based on observations on numerous experiments in which polyepi polymers were functionalized with various amines, the greater the number of amines attached to the PEi polymer, the greater the water solubility. Solutions of >50% by weight PEi / EDA have been achieved at ambient temperatures.
B. The procedure for the determination of the molecular weight involves gel permeation chromatography. For the determination of polyepichlorohydrin before any derivatization is performed, a PL-gel Mixed E column is used with tetrahydrofuran used as the solvent for the sample and as the eluent. Calibration was provided by comparison to commercial polyethylene glycol standards from Polymer Laboratories. Flow rates are controlled at 1 ml/min with a column temperature of 40°C. Samples are dissolved in tetrahydrofuran at a concentration of 0.25 weight percent and filtered to remove any particulates (which may include some very high molecular weight polymer). A loop injector is used to inject 150 microliters of solution onto the column. The resulting chromatograms are used to determine Mₙ, M_{w}, M_{z}, and M_{z+1} by mathematical calculation with software on the computer-controller. All molecular weights reported will represent M_{w} measurements.

Measurement of the molecular weight of the derivatized polyepichlorohydrin polymers was performed with a TSKgel 2000PW + 3000PW + 5000PW column using 0.1 M NaCl, 0.1 M EDA in 1 to 1 methanol/water at 1 ml/min and at a column temperature of 40°C. Injection volume was 100 microliters. Samples were dissolved in water at 1% concentration and filtered prior to injection. M_{w} values are reported.

The invention will be further clarified by a consideration of the following examples, which are intended to be purely exemplary of the present invention.

### EXAMPLES

### Starting Materials

### Example A: Preparation of Polyepichlorohydrin (PEi) Using Triethyloxionium Hexafluorophosphate.

In a dry atmosphere, 0.1257 g triethyloxionium hexafluorophosphate was dissolved in 9.4438 g dry methylene chloride. Distilled epichlorohydrin (78.4 g) was placed in a vessel flushed with dry nitrogen and immersed in a constant temperature bath at 40°C. The triethyloxionium hexafluorophosphate solution was added to the epichlorohydrin with stirring and allowed to react for twenty-four hours. The temperature was raised to 70°C when the reaction mixture became more viscous. The resulting material was rinsed with ethanol three times. Forty-eight grams of material were obtained. Gel permeation chromatography revealed a molecular weight range of 3,000 to 400,000 Daltons, with a median molecular weight (M_{w}) of 100,000 and 90% of the polymer having molecular weights between 5,000 and 100,000 Daltons.

### Example B: Preparation of Polyepichlorohydrin (PEi) Using Fluoboric Acid.

In a dry atmosphere, 450 mL of methylene chloride, 1.0 ml of 48% aqueous fluoboric acid, and 10 mL of 54% fluoboric acid in diethyl ether were heated to 40°C. To this composition 850 mL of epichlorohydrin was added slowly and refluxed until the reaction was complete. The reaction was evaporated with a rotary evaporator under reduced pressure and temperatures up to 100°C until no further solvent could be removed. The molecular weight of the polymer by gel permeation chromatography was 3500 Daltons (Mₙ) with over 40% of the material above 14,000 Daltons.

### Final Products

### Example 1: Preparation of Polyepichlorohydrin / Trimethylamine (PEi / TMA).

A 2 L stainless-steel, PARR pressure reactor was loaded with 185 g (2 moles) of polyepichlorohydrin polymer of molecular weight above 5,000 Daltons (FW 92.53 per repeating monomer unit). To this polyepichlorohydrin polymer was added 246.5 g (1 mole) of a 24% by weight solution of trimethylamine (FW 59.11). The reactor was sealed and placed in the PARR heater/stirrer unit and pressurized to 75 psi (Pa) with nitrogen. The reaction vessel was heated to 115°C with constant stirring. The reactor was maintained at 115°C and 75 psi (Pa) for sixteen hours. The reactor was cooled, vented to atmospheric pressure and opened. The reaction solution was filtered through a No. 1 filter paper on a 9.0 cm Buchner filter under vacuum, then transferred to a 500 mL round-bottomed flask. This solution was rotavaporated at 70°C and 8 inches (20.32 cm) of water vacuum pressure to 80 mL volume. This reaction product was transferred to a Spectra/Por^{™} membrane bag [molecular weight cutoff 14,000] and dialyzed in ten inches (25.4 cm) of deionized water for sixteen hours to remove any unreacted small molecular weight species. Molecular weight was about 18,000 Daltons (M_{w}).

### Example 2: Preparation of Polyepichlorohydrin / Trimethylamine / Ammonium hydroxide (PEi / TMA / NH₄OH).

A 2 L stainless-steel, PARR pressure reactor was loaded with 23.6 g (0.25 moles) of polyepichlorohydrin polymer (FW 92.53 per repeating monomer unit). To this polyepichlorohydrin polymer was added 250 mL of water and 30.8 g (0.125 moles) of a 24% by weight solution of trimethylamine (FW 59.11). The reactor was sealed and placed in the PARR heater/stirrer unit and heated to 105°C with constant stirring. The reactor was maintained at 105°C and 50 psi (Pa) for sixteen hours. The reactor was cooled, vented to atmospheric pressure and loaded with 450 g (7.7 moles) of 29% by weight ammonium hydroxide solution (FW 17). The reactor was resealed and placed in the heater/stirrer unit and reheated to 105°C. The reactor was maintained at 105°C and 80 psi (Pa) for sixteen hours. The reactor was then cooled, vented, and opened. The reaction solution was filtered through a No. 1 filter paper on a 9.0 cm Buchner filter under vacuum then transferred to a 500 mL round-bottomed flask. This solution was rotavaporated at 70°C and 23 inches (58.42 cm) of water vacuum pressure to 80 mL volume. This reaction product was transferred to a Spectra/Por^{™} membrane bag [molecular weight cutoff 3,500] and dialyzed in ten inches (25.4 cm) of deionized water for eighteen hours. This solution was then lyophilized to a light tan-colored, hygroscopic solid.

### Example 3: Preparation of Polyepichlorohydrin / Diethylenetriamine PEi / DETA).

A 500 mL, three-necked round bottomed flask was fitted with a reflux condenser, a thermometer to which a THERMOWATCH I²R temperature controller was attached, and an addition funnel. The flask was charged with 412.7 g (4 mole) of diethylenetriamine (FW 103.2), and then heated to 120°C. An addition funnel was charged with 37.7 g (0.41 mole) of polyepichlorohydrin with a molecular weight over 5,000 (monomer wt 92.53 g). The polyepichlorohydrin was added to the diethylenetriamine at a rate of about 0.25 mL per minute followed by continued heating of the reaction mixture for an additional 60 minutes and the cooling to 45°C. Sodium hydroxide solution (32.8 g, 0.41 mole) of 50% solution and 150 mL of water was mixed with the reaction mixture and stirred for 45 minutes, filtered with filter paper to remove a white precipitate, and dialyzed with Spectra/Por^{™} membrane with molecular weight cutoff of 3,500 Daltons. Solutions were then lyophilized to produce white powdered materials, having an average molecular weight of about 18,000 Daltons (M_{w}).

### Example 4: Preparation of Polyepichlorohydrin / Ethylenediamine (PEi / EDA).

A 2000 mL three-necked, round-bottomed flask was fitted with a stir bar, a reflux condenser, a 10 mL addition funnel and a thermometer to which a THERMOWATCH I²R temperature controller was attached. The flask was loaded with 360 g (6 moles) of ethylenediamine (EDA), (FW 60.1). The addition funnel was loaded with 231 g (2.5 moles) of polyepichlorohydrin polymer (FW 92.53 per repeating monomer unit) with a molecular weight above 5,000 Daltons and about 40% of the molecules above 12,000 Daltons. The reaction flask containing the EDA was heated to reflux (100°C) with constant stirring at which point the polyepichlorohydrin polymer was added dropwise to the ethylenediamine at a rate of about 4.5 mL per minute. The reaction was continued for 16 hours following the addition of all the polyepichlorohydrin polymer. The reaction mixture was then transferred to a round-bottomed flask and rotavaporated at 75°C and 23 inches (58.42 cm) of water vacuum pressure to remove unreacted ethylenediamine. The polyepichlorohydrin/EDA solution was transferred to a Spectra/Por^{™} membrane bag [molecular weight cutoff 14,000] and dialyzed in ten inches of deionized water for eighteen hours. This solution was then lyophilized to a light tan-colored, hygroscopic solid. Gel permeation chromatography revealed an average molecular weight, of over 17,000 Daltons (M_{w}).

### Comparative Example D: Preparation of Polyallylaminebiguanide (PAAG).

In a dry beaker, 9.36 g of polyallylamine hydrochloride (0.1 mole, purchased from Aldrich with a molecular weight of 50,000 to 65,000 Daltons) were mixed with 20 mL of 10M NaOH and sufficient water to allow stirring. The liquid was decanted and the resin was washed with water and dried. The resin was suspended in 300 mL of methanol in a round bottom flask and mixed with 20.12 g of 3,5-dimethylpyrazole-1-carboxamidine nitrate (0.1 mole). The reaction mixture was refluxed for 96 hours. The resin was then filtered and rinsed with methanol and dried. The product has a molecular weight of above about 75,000 Daltons.

### Comparative Example E: Preparation of Poly(allylamine-N-(2-hydroxy-3-trimethylammonium propyl) chloride).

Polyallyamine with molecular weight of 52,000 to 83,000 Daltons (1.88 g, 0.02 mole) was placed in a reaction vessel and mixed with 40.4548 g of 3M NaOH (0.121 mole). N, N, N-trimethyl-oxiranemethanaminium chloride (20.0150 g of 65.2% solution, 0.086 mole) was added. The reaction mixture was refluxed overnight and dialyzed in a 3,500 molecular weight cutoff dialysis bag against deionized water. The resultant solution was lyophilized, resulting in 1.8 g of tan solid. The solid is: 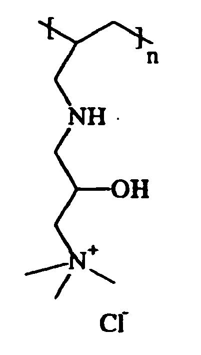 and has a molecular weight of above 75,000 Daltons.

### Comparative Example F: Preparation of Poly(allyl-N,N-dimethylamino-N-(2-hydroxy-3-trimethylammonium propyl) chloride).

Polyallyamine with molecular weight of 52,000 to 83,000 Daltons (0.9356 g, 0.01 mole) was dissolved in 5 g acetonitrile and reacted with 3.6 mL of 3M NaOH (0.0108 mole) to obtain a pH of 7.4. Methyl iodide (2.84 g, 0.02 mole) was added and the reaction mixture was refluxed. During reflux, an additional 0.8 mL of 3M NaOH (0.0024 mole) was added to raise the pH tc 7.9, N, N, N-trimethyl-oxiranemethanaminium chloride (5.77 g of 65.2% solution, 0.025 mole) was added and reflux was continued. After 24 hours, the reaction mixture was placed in a 3,500 Dalton cutoff dialysis bag and dialyzed overnight in deionized water. The resulting solution was lyophilized, resulting in 1.56 g (58% yield) of a tan powder. The compounc is: 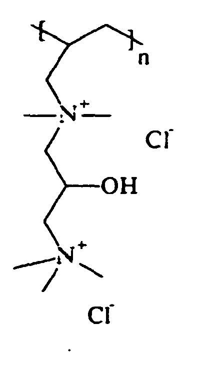 having a molecular weight of above 75,000 Daltons.

### Biological Examples

### Example I: Prevention of Gastrointestinal Phosphate Absorption by Use of Polyepichlorohydrin / Ethylenediamine.

Rat chow containing 0.65 gm% phosphorus from mixed grains was mixed with polyepichlorohydrin / ethylenediamine, prepared by the procedure of Example 4. Feed was prepared by mixing enough polyepichlorohydrin / ethylenediamine with the rat chow to have 3 moles of binding sites per mole of phosphate In the chow, 1 mole of binding sites per mole of phosphate in the chow, 0.5 mole of binding sites per mole of phosphate in the chow, and 0 moles of binding sites per mole of phosphate in the chow (the control group). Six rats were fed each diet for one week. During these experiments, the urinary phosphate at the end of the week averaged 18.2 mg/day for the controls, 8.7 mg/day in the 0.5X group, 8.6 mg/day in the 1X group, and 1.9 mg/day in the 3X group. The lower urinary phosphate levels indicate that the rats were reacting to not getting enough dietary phosphate by conserving phosphate through limiting renal excretion. The total phosphate balance (dietary intake - urinary output - stool output) at the end of the week was 47.8 mg/day for the controls, 50.9 mg/day for the 0.5X group, 34.3 mg/day for the 1X group, and 39.5 mg/day for the 3X group.

### Comparative Example A: Prevention of Gastrointestinal Absorption of Phosphate by Use of Polyallylamine (RenaStat^{™}).

Polyallylamine hydrochloride with a molecular weight of 50,000 to 65,000 was obtained from Aldrich and used without further purification. Rat chow containing 0.65 gm% phosphorus from mixed grains was mixed with polyallylamine hydrochloride in a ratio of 98.04 grams of powdered rat chow to 1.96 grams of polymer to provide a ratio of one amine binding site for each phosphate present in the feed. Two 125 g rats were fed *ad libitum* with this diet and compared to two rats fed *ad libitum* with unaltered powdered rat chow. Before starting the special diet and after a period of two weeks of stabilization, twenty four hour separate collections of stool and urine were obtained from each set of rats and analyzed for phosphorus by inductively coupled plasma spectroscopy. As the control rats decreased their growth rate over the two weeks, the percentage of the dietary phosphorus found in the stool increased from 65% to 72% (7% increase) while the rats on polyallylamine hydrochloride showed an increase from 58% to 75% (17% increase) of the dietary phosphorus being malabsorbed, which is 2.6 times the increased phosphate loss found in the control rats. During the same time, the control rats showed an increase in urinary phosphorus from 6% to 16% of the dietary intake while the rats on polyallylamine hydrochloride decreased their urinary phosphate output from 6% to 2% of the dietary intake of phosphate at the end of the study. This indicates that the rats on polyallylamine were retaining urinary phosphorus compared to the control rats indicating that they were unable to absorb adequate phosphate from their diet.

### Example II and Comparative Example A: Prevention of Gastrointestinal Phosphate Absorption with Polyallylaminebiguanide.

Two 125 g Sprague Dawley were given oral Nulytely^{™} to remove all material from their gastrointestinal tracts. After this preparation, one of the rats was given a gavage feeding of milk designed to deliver 0.324 millimole (mmol) phosphate. The other rat was gavage fed with the same amount of milk mixed with 0.0322 g or the polyallyaminebiguanide prepared in Example 5. After 1 hour, both rats were again given Nulytely^{™} to remove and collect all unabsorbed food from the gastrointestinal tract. Phosphate was measured in the collected feces by inductively coupled plasma spectroscopy. The phosphate that was malabsorbed by the rat that received the polyallyaminebiguanide was 66% of the phosphate malabsorbed by the control rat.

### Example III and Comparative Example B: Complexation of phosphate by Poly(allylamine-N-(2-hydroxy-3-trimethylammonium propyl) chloride).

Poly(allylamine-N-(2-hydroxy-3-trimethylammonium propyl) chloride), prepared by the procedure from Example 6, was dissolved in water as 0.72 g in 10 mL to make a 0.345 M solution. In each of four tubes, 0.54 mL of this solution (0.19 mmol of monomer units) were mixed with 10 mL of 0.0207 M NaH₂PO₄ and appropriate amounts of HCl and NaOH to make solutions of pH 3, pH 4.5, pH 6, and pH 7.5. Tubes with the same pH were also prepared with 10 mL of 0.0207 M NaH₂PO₄ and 0.50 mL of 1.5 M CaCO₃ (0.749 mmol). Control tubes were also prepared with the sodium phosphate solution and the adjustment of pH. All tubes were diluted to 12 mL with water. The tubes were agitated for one hour. The solutions were then placed in separate Centricon^{™} 30 molecular weight cutoff tubes and centrifuged for 30 minutes. The filtrate collected was analyzed for phosphorus by inductively coupled plasma spectroscopy. The polymer removed 58% of the phosphorus at pH 3, 59% of the phosphorus at pH 4.5, 56% of the phosphorus at pH 6, and 44% of the phosphorus at pH 7.5. The calcium carbonate removed 16% of the phosphorus at pH 3, 13% of the phosphorus at pH 4.5, 9% of the phosphorus at pH 6, and 7% of the phosphorus at pH 7.5. The control tubes showed 0.7% of the phosphorus removed at pH 3, 1.1% of the phosphorus removed at pH 4.5, 0.4% of the phosphorus removed at pH 6, and 0.6% of the phosphorus removed at pH 7.5. Thus, the poly(allylamine-N-(2-hydroxy-3-trimethylammonium propyl) chloride) was effective in complexing phosphate.

### Example IV and Comparative Example C: Complexation of phosphate by Poly(allyl-N,N-dimethylamino-N-(2-hydroxy-3-trimethylammonium propyl) chloride).

Poly(allyl-N,N-dimethylamino-N-(2-hydroxy-3-trimethytammonium propyl) chloride) as prepared in Example 7 was dissolved in water as 0.72 g in 10 mL to make a 0.263 M solution. In each of four tubes, 0.67 mL of this solution (0.18 mmol of monomer units) were mixed with 10 mL of 0.0207 M NaH₂PO₄ and appropriate amounts of HCl and NaOH to make solutions of pH 3, pH 4.5, pH 6, and pH 7.5. Tubes with the same pH were also prepared with 10 mL of 0.0207 M NaH₂PO₄ and 0.50 mL of 1.5 M CaCO₃ (0.749 mmol). Control tubes were also prepared with the sodium phosphate solution and the adjustment of pH. All tubes were diluted to 12 mL with water. The tubes were agitated for one hour. The solutions were then placed in separate Centricon^{™} 30 molecular weight cutoff tubes and centrifuged for 30 minutes. The filtrate collected was analyzed for phosphorus by inductively coupled plasma spectroscopy. The polymer removed 49% of the phosphorus at pH 3, 53% of the phosphorus at pH 4.5, 48% of the phosphorus at pH 6, and 39% of the phosphorus at pH 7.5. The calcium carbonate removed 16% of the phosphorus at pH 3, 13% of the phosphorus at pH 4.5, 9% of the phosphorus at pH 6, and 7% of the phosphorus at pH 7.5. The control tubes showed 0.7% of the phosphorus removed at pH 3, 1.1% of the phosphorus removed at pH 4.5, 0.4% of the phosphorus removed at pH 6, and 0.6% of the phosphorus removed at pH 7.5. Thus, the poly(allyl-N,N-dimethylamino-N-(2-hydroxy-3-trimethylammonium propyl) chloride) was effective in complexing phosphate.

### Example V: Complexation of oxalate by Polyepichlorohydrin / EDA and Polyepichlorohydrin /DETA.

An 0.025M solution of ammonium oxalate was prepared. Polyepichlorohydrin was prepared using the procedure from Example A, having a molecular weight of about 45,000 Daltons. The EDA (Example 4 above) and DETA (Example 3 above) derivatives were made. Solutions of these two derivatives were used to place 0.001 mole of the binding sites into separate molecular weight cutoff filters (Centricon^{™} concentrators), then 0.001 mole of oxalate was added to each concentrator and to a control containing only water. The solutions were mixed together for an hour then centrifuged. The filtrates were analyzed for oxalate by GC-MS and compared with the concentrator which had only oxalate. Both EDA and DETA derivative polymers had absorbed about 30% of the oxalate.

### Example VI: Prevention of Gastrointestinal Phosphate Absorption by Use of Polyepichlorohydrin / Trimethylamine / Ammonia.

Rat chow containing 0.65 gm% phosphorus from mixed grains was mixed with polyepichlorohydrin / trimethylamine / ammonia prepared in Example 2 in a ratio of 97.64 grams of powdered rat chow to 2.36 grams of polymer to provide a ratio of one amine binding site for each phosphate present in the feed. Two 125 g rats were fed *ad libitum* with this diet and compared to two rats fed *ad libitum* with unaltered powdered rat chow. Before starting the special diet and after a period of two weeks of stabilization, twenty four hour separate collections of stool and urine were obtained from each set of rats and analyzed for phosphorus by inductively coupled plasma spectroscopy. As the control rats decreased their growth rate over the two weeks, the percentage of the dietary phosphorus found in the stool increased from 65% to 72% (7% increase) while the rats on polyepichlorohydrin /trimethylamine / ammonia showed an increase from 65% to 76% (11% increase) of the dietary phosphorus being malabsorbed, which is 1.6 times the increased phosphate loss found in the control rats. During the same time, the control rats showed an increase in urinary phosphorus from 6% to 16% of the dietary intake while the rats on polyepichlorohydrin / trimethylamine / ammonia had 7% of the dietary phosphorus in their urine on the normal diet and 10% of the dietary phosphorus in the urine at the end of the study. Thus, the rats on polyepichlorohydrin / trimethylamine / ammonia were retaining phosphorus compared to the control rats, indicating that they were not able to absorb adequate amounts of phosphate from their food.

### Example VII: Prevention of Gastrointestinal Phosphate Absorption by Use of Polyepichlorohydrin / Trimethylamine.

Rat chow containing 0.65 gm% phosphorus from mixed grains was mixed with polyepichlorohydrin / trimethylamine prepared in Example 1 in a ratio of 96.82 grams of powdered rat chow to 3.18 grams of polymer to provide a ratio of one amine binding site for each phosphate present in the feed. Two 125 g rats were fed *ad libitum* with this diet and compared to two rats fed *ad libitum* with unaltered powdered rat chow. Before starting the special diet and after a period of two weeks of stabilization, twenty four hour separate collections of stool and urine were obtained from each set of rats and analyzed for phosphorus by inductively coupled plasma spectroscopy. As the control rats decreased their growth rate over the two weeks, the percentage of the dietary phosphorus found in the stool increased from 65% to 72% (7% increase) while the rats on polyepichlorohydrin /trimethylamine showed an decrease from 62% to 59% (3% decrease) of the dietary phosphorus being malabsorbed. During the same time, the control rats showed an increase in urinary phosphorus from 6% to 16% (10% increase) of the dietary intake while the rats on polyepichlorohydrin / trimethylamine had 6% of the dietary phosphorus in their urine on the normal diet and 9% of the dietary phosphorus in the urine at the end of the study (3% increase). This modest increase indicates that the rats on polyepichlorohydrin /trimethylamine were retaining phosphorus compared to the control rats.

Other embodiments of the invention will be apparent to those skilled in the art from a consideration of this specification or practice of the invention disclosed herein. It is intended that the specification and examples be considered as exemplary only, with the true scope and spirit of the invention being indicated by the following claims.

## Claims

1. A formulation for oral administration, comprising a water-soluble polyether glycol polymer and a pharmaceutically-acceptable carrier, wherein the polymer comprises a structural backbone of carbon atoms and oxygen atoms where there are at least two consecutive carbon atoms present between each oxygen atom; a moiety on the backbone of the polymer or a functionalised derivative on the polymer, that is cationic at physiological pH and permits complex formation with phosphate or oxalate, a weight average molecular weight (M_{w}) from 5,000 to 80,000 Daltons, and wherein the solubility of the polymer is at least 0.01 gram of the polymer per 1,000 mL of water.

2. The composition of Claim 1 wherein the polymer has an average molecular weight from 10,000 to 80,000 Daltons.

3. The composition of Claim 2 wherein the polymer has an average molecular weight from 12,000 to 80,000 Daltons.

4. The composition of Claim 2 wherein the polymer has an average molecular weight from 15,000 to 80,000 Daltons.

5. The composition of Claim 1 wherein the polymer is derivatised with functional groups.

6. The composition of Claim 5 wherein the functional groups are either directly connected to the polymer backbone or connected through C₂-C₆ alkylene or C₂-C₅ alky-C₆-C₁₂-aryl groups, and are selected from amine, and phosphine, or combinations of these groups.

7. The composition of Claim 6 wherein the polymer is a polyepihalohydrin derivative.

8. The composition of Claim 7 wherein the polyepihalohydrin derivative has an average molecular weight of between 15,000 to 80,000 Daltons.

9. The composition of Claim 7 wherein the polyepihalohydrin derivative is polyepichlorohydrin amine.

10. The composition of Claim 9 wherein the derivative is a trimethylamine group.

11. The composition of Claim 9 wherein the derivative is a triethylenetetramine group.

12. The composition of Claim 9 wherein the derivative is an ethylenediamine group.

13. The composition of Claim 9 wherein the derivative is a diethylenetriamine group.

14. The composition of Claim 9 wherein the derivative is a tetraethylenepentamine group.

15. The composition of Claim 9 wherein the derivative is a mixture of two or more amine groups.

16. The composition of Claim 1 wherein the solubility of the polymer is from 1 to 10 grams of polymer per 1 mL of water.

17. The use of a water-soluble polymer in the preparation of a pharmaceutical composition for the reduction of phosphate or oxalate in vivo in an animal, wherein the polymer is a polymer as defined in any one of Claims 1 to 16.

18. A process for preparing a composition as claimed in any one of Claims 1 to 16, which process includes the step of preparing the water-soluble polymer, by reacting an epihalohydrin in the presence of a Lewis acid of moderate strength, in a solvent that will not act as a chain terminator.

19. The process of Claim 18 wherein the solvent is dichloromethane.

20. A process for preparing a composition as claimed in Claim 1 which process includes the step of preparing the water-soluble polymer, by reacting a 3,4- dichloro-1 2-butane oxirane, in the presence of a Lewis acid of moderate strength, in a solvent that will not act as a chain terminator.

21. A process as claimed in Claim 18 or Claim 20, wherein a catalyst is present selected from triethyloxionium hexafluorophosphate, fluoboric acid, triethyl aluminum, and 1,2-ethyl di(trifluoromethanesulfonate).

## Patentansprüche

1. Formulierung zur oralen Verabreichung, umfassend ein wasserlösliches Polyetherglycol-Polymer und einen pharmazeutisch annehmbaren Träger, wobei das Polymer ein strukturelles Rückgrat aus Kohlenstoffatomen und Sauerstoffatomen umfasst, wobei zwischen den Sauerstoffatomen jeweils wenigstens zwei aufeinander folgende Kohlenstoffatome vorhanden sind; eine Gruppe an dem Rückgrat des Polymers oder ein funktionalisiertes Derivat an dem Polymer, das bei physiologischem pH-Wert kationisch ist und eine Komplexbildung mit Phosphat oder Oxalat erlaubt, ein gewichtsmittleres Molekulargewicht (M_{w}) von 5 000 bis 80 000 Dalton und wobei die Löslichkeit des Polymers wenigstens 0,01 Gramm Polymer pro 1 000 ml Wasser beträgt.

2. Zusammensetzung nach Anspruch 1, wobei das Polymer ein durchschnittliches Molekulargewicht von 10 000 bis 80 000 Dalton aufweist.

3. Zusammensetzung nach Anspruch 2, wobei das Polymer ein durchschnittliches Molekulargewicht von 12 000 bis 80 000 Dalton aufweist.

4. Zusammensetzung nach Anspruch 2, wobei das Polymer ein durchschnittliches Molekulargewicht von 15 000 bis 80 000 Dalton aufweist.

5. Zusammensetzung nach Anspruch 1, wobei das Polymer mit funktionellen Gruppen derivatisiert ist.

6. Zusammensetzung nach Anspruch 5, wobei die funktionellen Gruppen entweder direkt an das Polymerrückgrat gebunden sind oder über C₂-C₆-Alkylen- oder C₂-C₅-Alkyl-C₆-C₁₂- Arylgruppen gebunden sind, und sie aus Amin und Phosphin oder Kombinationen dieser Gruppen ausgewählt sind.

7. Zusammensetzung nach Anspruch 6, wobei das Polymer ein Polyepihalohydrin-Derivat ist.

8. Zusammensetzung nach Anspruch 7, wobei das Polyepihalohydrin-Derivat ein durchschnittliches Molekulargewicht von zwischen 15 000 und 80 000 Dalton aufweist.

9. Zusammensetzung nach Anspruch 7, wobei das Polyepihalohydrin-Derivat ein Polyepichlorhydrinamin ist.

10. Zusammensetzung nach Anspruch 9, wobei das Derivat eine Trimethylamin-Gruppe ist.

11. Zusammensetzung nach Anspruch 9, wobei das Derivat eine Triethylentetramin-Gruppe ist.

12. Zusammensetzung nach Anspruch 9, wobei das Derivat eine Ethylendiamin-Gruppe ist.

13. Zusammensetzung nach Anspruch 9, wobei das Derivat eine Diethylentriamin-Gruppe ist.

14. Zusammensetzung nach Anspruch 9, wobei das Derivat eine Tetraethylenpentamin-Gruppe ist.

15. Zusammensetzung nach Anspruch 9, wobei das Derivat ein Gemisch aus zwei oder mehr Amingruppen ist.

16. Zusammensetzung nach Anspruch 1, wobei die Löslichkeit des Polymers von 1 bis 10 Gramm Polymer pro 1 ml Wasser beträgt.

17. Verwendung eines wasserlöslichen Polymers bei der Herstellung einer pharmazeutischen Zusammensetzung zur Verringerung der in vivo Phosphat- oder Oxalat-Werte in einem Tier, wobei das Polymer ein wie in einem der Ansprüche 1 bis 16 definiertes Polymer ist.

18. Verfahren zur Herstellung einer Zusammensetzung wie in einem der Ansprüche 1 bis 16 definiert, wobei das Verfahren den Schritt der Herstellung des wasserlöslichen Polymers einschließt, indem ein Epihalohydrin in Gegenwart einer mittelstarken Lewissäure in einem Lösungsmittel das nicht als Kettenterminator wirkt umgesetzt wird.

19. Verfahren nach Anspruch 18, wobei das Lösungsmittel Dichlormethan ist.

20. Verfahren zur Herstellung einer Zusammensetzung nach Anspruch 1, wobei das Verfahren den Schritt der Herstellung des wasserlöslichen Polymers einschließt, indem ein 3,4-Dichlor-1,2-butanoxiran in Gegenwart einer mittelstarken Lewissäure in einem Lösungsmittel das nicht als Kettenterminator wirkt umgesetzt wird.

21. Verfahren nach Anspruch 18 oder Anspruch 20, wobei ein Katalysator zugegen ist, der ausgewählt ist aus Triethyloxonium-Hexafluorophosphat, Fluorborsäure, Triethylaluminium und 1,2-Ethyl-di(trifluormethansulfonat).

## Revendications

1. Formulation pour une administration par voie orale, comprenant un polymère de polyéther glycol hydrosoluble et un support pharmaceutiquement acceptable, dans laquelle le polymère comprend un squelette structurel d'atomes de carbone et d'atomes d'oxygène où il y a au moins deux atomes de carbone consécutifs présents entre chaque atome d'oxygène ; un fragment sur le squelette du polymère ou un dérivé fonctionnalisé sur le polymère, qui est cationique au pH physiologique et permet la formation d'un complexe avec du phosphate ou de l'oxalate, un poids moléculaire moyen en poids (Mm) de 5 000 à 80 000 daltons, et dans laquelle la solubilité du polymère est d'au moins 0,01 g du polymère pour 1 000 ml d'eau.

2. Composition selon la revendication 1, dans laquelle le polymère a un poids moléculaire moyen de 10 000 à 80 000 daltons.

3. Composition selon la revendication 2, dans laquelle le polymère a un poids moléculaire moyen de 12 000 à 80 000 daltons.

4. Composition selon la revendication 2, dans laquelle le polymère a un poids moléculaire moyen de 15 000 à 80 000 daltons.

5. Composition selon la revendication 1, dans laquelle le polymère est dérivé avec des groupes fonctionnels.

6. Composition selon la revendication 5, dans laquelle les groupes fonctionnels sont soit directement liés au squelette du polymère, soit liés par des groupes alkylènes en C₂-C₆ ou C₂-C₅ alkyle-C₆-C₁₂ aryle, et sont choisis parmi une aminé, et une phosphine, ou des combinaisons de ces groupes.

7. Composition selon la revendication 6, dans laquelle le polymère est un dérivé de la polyépihalohydrine.

8. Composition selon la revendication 7, dans laquelle le dérivé de la polyépihalohydrine à un poids moléculaire moyen entre 15 000 et 80 000 daltons.

9. Composition selon la revendication 7, dans laquelle le dérivé de la polyépihalohydrine est l'amine polyépichlorhydrine.

10. Composition selon la revendication 9, dans laquelle le dérivé est un groupe triméthylamine.

11. composition selon la revendication 9, dans laquelle le dérivé est un groupe triéthylènetétramine.

12. Composition selon la revendication 9, dans laquelle le dérivé est un groupe éthylènediamine.

13. Composition selon la revendication 9, dans laquelle le dérivé est un groupe diéthylènetriamine.

14. Composition selon la revendication 9, dans laquelle le dérivé est un groupe tétraéthylènepentamine.

15. Composition selon la revendication 9, dans laquelle le dérivé est un mélange de deux groupes amines ou plus.

16. Composition selon la revendication 1, dans laquelle la solubilité du polymère est de 1 à 1 g de polymère pour 10 ml d'eau.

17. Utilisation d'un polymère hydrosoluble dans la préparation d'une composition pharmaceutique destinée à la réduction de phosphate ou d'oxalate in vivo chez un animal, dans laquelle le polymère est un polymère tel que défini dans l'une quelconque des revendications 1 à 16.

18. Procédé de préparation d'une composition selon l'une quelconque des revendications 1 à 16, lequel procédé comprend l'étape de préparation du polymère hydrosoluble, en faisant réagir une épihalohydrine en présence d'un acide de Lewis de force modérée, dans un solvant qui ne va pas agir comme terminateur de chaîne.

19. Procédé selon la revendication 18, dans lequel le solvant est le dichlorométhane.

20. Procédé de préparation d'une composition selon la revendication 1, lequel procédé comprend l'étape de préparation du polymère hydrosoluble, en faisant réagir un 3,4-dichloro-1,2-butane-oxirane, en présence d'un acide de Lewis de force modérée, dans un solvant qui ne va pas agir comme terminateur de chaîne.

21. procédé selon la revendication 18 ou la revendication 20, dans lequel un catalyseur est présent et est choisi parmi l'hexafluorophosphate de triéthyloxionium, l'acide fluoborique, le triéthylaluminium et le 1,2-éthyle di(trifluorométhanesulfonate).
